# EUROPEAN PATENT SPECIFICATION

(11) **EP 2 815 739 B1**
(45) Date of publication and mention of the grant of the patent: **28.08.2019**
(21) Application number: 14172486.4
(22) Date of filing: 16.06.2014
(51) Int. Cl.: A61K 9/00, A61K 9/14, A61K 9/16, A61K 9/48, A61K 31/56

(54) **Inhalation composition filling method**
Inhalationszusammensetzungs-Füllverfahren
Procédé de remplissage de composition d'inhalation

(30) Priority: 17.06.2013 TR 201307244
(43) Date of publication of application: 24.12.2014
(73) Proprietor: Arven Ilac Sanayi Ve Ticaret A.S., Istanbul 34460 (TR)
(72) Inventor: Türkyilmaz, Ali, 34460 Istanbul (TR); Celik, Devrim, 34460 Istanbul (TR); Özölmez, Levent, 34460 Istanbul (TR); Mutlu, Onur, 34460 Istanbul (TR)
(74) Representative: Sevinç, Erkan

(56) References cited:
- EP-A1- 2 221 048
- WO-A2-2011/093818
- US-A1- 2010 236 550

## Description

### Field of Invention

The present invention relates to pharmaceutical powder compositions administered using inhalation devices. The present invention particularly relates to a filling method which provides an ideal volume and density for the compositions used in inhalation devices and delivers a desired amount of a fine particle dose to the patient.

### Background of Invention

Fluticasone is a synthetic corticosteroid of medium potency. Its chemical designation is 6α,9-difluoro-17{[(fluoromethyl)sulfanyl]carbonyl}-11β-hydroxy-16α-methyl-3-oxoandrosta-1,4-dien-17α-yl furan-2-carboxylate with the following chemical structure of Formula I.

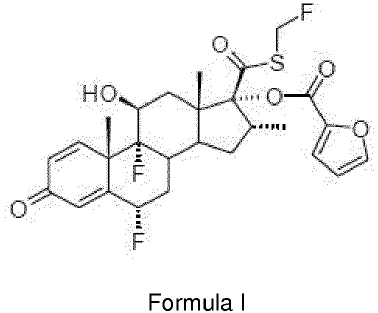

Fluticasone is used in the treatment of allergic rhinitis, asthma and chronic obstructive pulmonary disease. It is commercially available under the trademark Flixotide® in the form of 60 blister-inhaler devices each blister containing 100 mcg fluticasone propionate.

Fluticasone was originally disclosed in the patent US4335121.

Salmeterol is a highly-selective b2-agonist used via inhalation in the prophylaxis of bronchospasm in the adults with asthma and reversible obstructive respiratory tract disease. When compared to other b2-agonist drugs, it has a longer action time and allows to twice-daily administration. However, the use of salmeterol is limited because of the slow onset time of its therapeutic effect. Its chemical name is (RS)-5-{1-hydroxy-2-(6-(4-phenylbutoxy)-hexylamino)ethyl}salicyl alcohol 1-hydroxy-2-naphthoate. Its chemical structure is illustrated in Formula 2 given below.

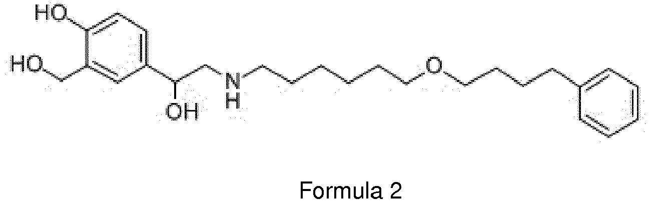

It is commercially available under the trademark Serevent®.

Salmeterol was originally disclosed in the patent US4992474.

In prior art, some studies exist to formulate dry powder inhalation formulations. For example, EP 2 221 048 A1 discloses a formulation for inhalation which aims to provide reduced interactions between active ingredients, better stability, homogeneity and higher bioavailability, lowering the amount of the active ingredients. The formulation proposed in EP 2 221 048 A1 involves a medicament fraction of at least two active ingredients and at least two pharmaceutically acceptable excipients, wherein the active ingredients are adhered to said excipients, and wherein each active ingredient is adhered to a different excipient, characterized in that the d50 value of the individual excipients differs by more than 10 %, preferably more than 15%, most preferably more than 20%.

Formulations of salmeterol and fluticasone combination are known from US 2010/236550 A1 and WO 2011/093818 A2.

US 2010/236550 A1 relates to a method in which the medicament is fractionated prior to formulation. The method involves (i) fractionating a particulate active ingredient based on aerodynamic particle size, (ii) recovering at least one fraction of the particulate active ingredient to obtain a recovered fraction and (iii) combining the recovered fraction with a carrier to provide the inhalable dry-powder medicament.

Dry powder inhalation formulations of salmeterol xinafoat and fluticasone propionate are disclosed in WO 2011/093818 A2. In order to ensure effective absorption of the active agents into the lung tissue, the particle size of the agents should be adjusted. The suggested solution involves dry powder formulation of salmeterol xinafoat and fluticasone propionate; wherein the mean particle size of active agents is in the range of 1.5 to 4.5 µm.

Inhalation compositions act by directly reaching the respiratory tract. The design of the compositions is based on comprising an active agent and a carrier having a particle size capable to carry the active agent to the respiratory tract and a filler. On the other hand, the size of the carrier particles providing the delivery of a desired level of active agent to the respiratory tract is also critical. it is determined that the flow properties of a composition and the filling of the composition are depended on particle sizes and its ratio. Having this proportion at a desired extent is quite critical, wherein the filling process rate and the amount of composition to be filled depend on this proportion. It is preferred to obtain a homogeneous mixture and to carry out the filling of this mixture by an economic method which is advantageous in terms of the process rate.

Furthermore, the content uniformity of a drug is a prerequisite with respect to user safety and treatment efficiency. The difference of the particle sizes of the carrier and of the filler used to provide content uniformity is significant. If this difference is excessive, no content uniformity can be ensured. Another potential problem is the lack of providing dose accuracy in terms of each cavity or capsule. This, in turn, is of vital importance in terms of treatment efficiency.

In order to meet all these requirements specified above, dry powder inhalers (DPI) are needed to fulfill a series of requisites, taking into account particularly the following parameters:

### Content uniformity of an active drug:

In a single dose system, each capsule or blister should contain the same amount of drug. In a multi dose system, in turn, it is required to release the same amount of drug in each administration to guarantee that a patient receives the same dose every time. The presence of a carrier should promote content uniformity even in a low-dose drug.

### Flowability:

The design of the device, the properties of the active agents and the filling platform to be used determine the required features of the carrier to be employed. The flow properties of a composition bears importance for guaranteeing that a device carries out the entire functions in a correct manner and provides an uninterrupted performance. Here, the selection of the carrier is very important for guaranteeing that the device works correctly and delivers a correct amount of active agent to the patient. For this reason, it is very important to use lactose, as the carrier, in two different particle sizes (fine and coarse).

### Dose consistency:

For guaranteeing that all doses released from the device contain a correct amount of active agent, the dry powder inhaler (DPI) devices should have a consistent dose uniformity. It is quite important that the dose released from the dry powder inhaler device is the same every time, irrespective of the inhalation ability of the patient. For this reason, using lactose as a carrier with correct properties in the composition helps in administering the dose in a consistent manner.

Small drug particles tend to agglomerate. This agglomeration can be prevented using suitable carrier or carrier mixtures. Additionally, they help in controlling the flowability of the drug being released from the device and providing a correct and consistent dosage of the active agent delivered to the lungs.

In addition, the mixture by which the drug particles bind to the carrier should be homogeneous. This binding, however, should not be too strong as the drug would not be released from the carrier particle during inhalation. It should also be possible to fill a low dose of powder to the device and the drug should be released in the same manner all the time. One of the main parameters for the composition is the particle size of the carrier. For this reason, using a correct proportion of fine and coarse particles of the carrier selected for the compositions according to the present invention was found to be of great importance.

In order to fulfill all these requirements, the compositions and particularly the carriers used in the compositions for dry powder inhaler (DPI) devices should be selected and adapted carefully. In order to fulfill these requirements, inhalable fine or microfine particles of active agents are mixed with carriers. By virtue of the mixing process, the particle size of the carrier can be changed so that some proportion of the carrier can be inhaled. The particle size of the carrier used is based on the requirements and specifications of the powder inhalator used for administering the composition. No decomposition should take place in these mixtures during the whole process, including transporting, storage, and dosing, namely, the active agent particles should not be separated from the carrier particles. However, as induced by the inhalation of the patient, the active agent particles should be separable as efficiently as possible, i.e. as much as possible, to be inhaled during the separation process in the inhaler.

Administering a drug vial inhalation can take place using devices of various types and sizes. Drugs having dosage amounts determined for use in these devices are stored in multi blisters or alternatively in capsules for providing convenience in single uses. The filling process and conditions of the drugs into blisters or capsules are of vital importance in the delivery of drug to the patient and in determining the target dosage amount to be delivered. The drug compositions are filled into blister cavities and capsules at a defined volume and weight proportion. As the filling process is carried out at machines, it is required to fit the compositions into the blisters or capsules. This, in turn, determines if a complete drug-delivery is achieved or not. For instance, the particle size and particle shape of the components in the composition directly influence the volume and accordingly the bulk density and compressed density. When the volume of the composition is high, the compositions may not be fitted into the capsules and blisters. Compressing the composition to provide the fit, in turn, may result in that a desired level of the fine particle dose amount cannot be delivered to the patient so that an underdose drug delivery takes place during administration.

In brief, while the finished composition is filled into capsules or blisters, it is compressed to prevent it from occupying an excessive volume and to bring it to certain bulk and compressed density values. However, this is a critically issue. If high pressure is exerted to the composition during the filling process, the breath of the user during administration may not provide a drug delivery at the exact dosage. As a result of this, desired results cannot be achieved in the treatment.

For this reason, a novel composition and filling process are required to overcome the aforesaid problems.

In result, a novelty is required in the art of compositions which can be used by patients having asthma and chronic obstructive pulmonary disease.

### Object and Brief Description of Invention

The present invention relates to easily-administrable inhalation compositions, eliminating all of the problems described above and bringing additional advantages to the relevant prior art.

Accordingly, the main object of the present invention is to provide a filling process guaranteeing the delivery of an exact dose of a composition to a patent by reaching target flow and NGI (device making fine particle dose measurement) values.

Another object of the present invention is to obtain a composition and package embodiment with an ideal volume, density, and content uniformity by virtue of a method having a suitable vacuum range.

A further object of the present invention is to provide a filling process which, on the one hand, facilitates the filling process into blisters or capsules, and on the other hand, makes it possible to realize a uniform mixture, by which inhalation compositions are obtained having a suitable particle size and proportions.

A further object of the present invention is to place the composition in a compressed state occupying a minimum volume in the cavity, and thus to achieve target NGI or fine particle dosages during administration.

A further object of the present invention is to realize a composition by which desired filling rates and content uniformity are achieved.

A filling method of an inhaler composition according to claim 1 is developed to achieve all objects referred to above and to be disclosed in the following detailed description.

In a preferred embodiment according to the present invention, the novelty is characterized in that a composition having a bulk density ranging from 0.48 to 0.56 g/mL, a compressed density ranging from 0.78 to 0.92 g/mL, a d(50) particle size ranging from 30 to 50 µm and comprising 0.2 to 1.7% by weight of salmeterol with a d(50) particle size distribution ranging from 1 to 3 µm, 0.2 to 17.0% by weight of fluticasone with a particle size distribution ranging from 1 to 3 µm, 1 to 20% by weight of fine particle lactose with a d(50) particle size ranging from 2 to 10 µm, and 80 to 99% by weight of coarse particle lactose with a d(50) particle size ranging from 50 to 120 µm, is taken into a reservoir by applying a vacuum of 50000 Pa (500mBar) to 8000 Pa (80 mBar) and then stopping the applied vacuum and transferring the composition into the blister cavity.

According to a preferred embodiment of the present invention, said reservoir is provided on a cylinder.

According to a preferred embodiment of the present invention, said cylinder further comprises additional reservoirs.

According to a preferred embodiment of the present invention, the composition taken into the reservoir under vacuum effect is filled in two parts into a blister or capsule.

According to a preferred embodiment of the present invention the bulk density of the composition is 0.50 - 0.55 g/mL.

According to a preferred embodiment of the present invention the compressed density of the composition is 0.80 - 0.90 g/mL.

According to a preferred embodiment of the present invention, the vacuum applied is preferably between 40000 Pa (400 mBar) - 10000 Pa (100 mBar).

### Examples

1 -

| | **50/100 mcg** | | | |
|---|---|---|---|---|
| **Content** | **5 mg** | **%** | **25 mg** | **%** |
| **Fluticasone** | 0,1 | 2 | 0,1 | 0,4 |
| **Salmeterol** | 0,05 | 1 | 0,05 | 0,2 |
| **Lactose** | 4,85 | 97 | 24,85 | 99,4 |
| **Total** | 5 | 100 | 25 | 100 |

2 -

| | **50/250 mcg** | | | |
|---|---|---|---|---|
| **Content** | **5 mg** | **%** | **25 mg** | **%** |
| **Fluticasone** | 0,25 | 5 | 0,25 | 1 |
| **Salmeterol** | 0,05 | 1 | 0,05 | 0,2 |
| **Lactose** | 4,7 | 94 | 24,7 | 98,8 |
| **Total** | 5 | 100 | 25 | 100 |

3 -

| | **50/500 mcg** | | | |
|---|---|---|---|---|
| **Content** | **5 mg** | **%** | **25 mg** | **%** |
| **Fluticasone** | 0,5 | 10 | 0,5 | 2 |
| **Salmeterol** | 0,05 | 1 | 0,05 | 0,2 |
| **Lactose** | 4,45 | 89 | 24,45 | 97,8 |
| **Total** | 5 | 100 | 25 | 100 |

The compositions according to the present invention comprising mixtures of fine particle-coarse particle lactose and active agents can be produced using the processes according to the prior art.

### Fine particle carriers (lactose):

d10; 1.0 - 5.0 µm or d10; 1.0 - 4.0 µm,
d50; 2.0 - 10.0 µm or d50; 4.0 - 7.0 µm,
d90; 7.0 - 20.0 µm or d90; 7.0 - 15.0 µm.

### Coarse particle carriers (lactose):

d10; 10.0 - 50.0 µm
d50; 50.0 - 120.0 µm or d50; 50.0 - 75.0 µm
d90; 120.0 - 300.0 µm or d90; 75.0 - 250.0 µm.

Particle size measurements were conduced by laser diffraction method using a Malvern Mastersizer 2000 device. The particle size is measured by volume. The preferred measurement method is wet dispersion.

According to the present invention, a composition having a bulk density ranging from 0.48 to 0.56 g/mL, a compressed density ranging from 0.78 to 0.92 g/mL, a d(50) particle size ranging from 30 to 50 µm and comprising 0.2 to 1.7% by weight of salmeterol with a d(50) particle size distribution ranging from 1 to 3 µm, 0.2 to 17.0% by weight of fluticasone with a particle size distribution ranging from 1 to 3 µm, 1 to 20% by weight of fine particle lactose with a d(50) particle size ranging from 2 to 10 µm, and 80 to 99% by weight of coarse particle lactose with a d(50) particle size ranging from 50 to 120 µm is taken into a reservoir by applying a vacuum of 50000 Pa (500 mBar) to 8000 Pa (80 mBar) and then the applied vacuum is terminated and the composition is transferred into the blister cavity. The invention carried out according to these values has certain compression limits. This is determined according to the proportions of the mixture and the particle size selected. The composition preferred according to the present invention is taken into a reservoir on the surface of a cylinder by means of a vacuum effect. Thus, the powder composition takes the form of the reservoir by being compressed to a controllable extent. Then, the vacuum application is interrupted and the shaped composition is taken into blisters or capsules. Here, the filling process of blisters or capsules is effected without giving rise to losses or wastes. Compressing the composition with determined vacuum applied which delivers a desired qualification and amount of drug to the patient. Compositions simultaneously taken into two cylinders arranged side by side and compressed accordingly can be placed into a single cavity or blister. The composition taken at the required dosage can be placed into blister cavities in two parts. Thus, the composition is fitted into the cavities and is delivered to the target site during inhalation.

These preferred values also substantially facilitate the flow and filling of the components during the process. Thus, a homogenous mixture is obtained and the filling of this mixture is realized in an economical and rapid manner.

Coarse carrier particles are used for preventing the (re-)agglomeration of the fine particles of the active agent. In order to achieve this effect, a carrier is used which has a particle size which is around 10 times larger than that of the active agent. Generally a single layer of the active agent particles is formed over the large carrier particles. Since the active agent and the carrier agent are to be separated from each other during inhalation, the shape and the surface roughness is particularly important. The particles of a carrier agent having a smooth surface will be separated from the particles of the active agent more easily as compared to the particles of a carrier agent of the same size, but having a porous surface structure.

Fine carrier particles are used for promoting the delivery of active agents to the lungs more safely in higher doses. In this context, since the surface energy is not evenly distributed over the carrier particle under normal conditions, the active agent tends to localize on the sites having a higher surface energy. This, in turn, makes it more difficult for the active agent to be released from the carrier agent particularly in low-dose compositions following pulmonary administration. Since high-energy regions will be covered by fine carrier particles and accordingly the active agent particles will tend to bind to low-energy regions, using fine carrier particles with a particle size below 10.0 micron or 5.0 micron will help in reducing this circumstance. It was discovered that increasing the fraction of the fine carrier particles enhances the pulmonary uptake. Accordingly, reducing the particle size (providing even finer particles) increases the fluidity and this, in turn, increases the amount of drug delivered to the lungs.

Drug particles will then bind to low-binding regions and will be more easily released during inhalation. Adding fine particles will substantially increase the surface area and reduce the carrying capacity. Using fine carrier particles having a slightly larger particle size than that of the drug particles may eliminate the friction forces between the drug and the carrier during the blending process.

Another object of the present invention is to adjust the flowability of compositions in a correct manner in order to guarantee that correct amounts of active agents are delivered by DPI devices. In other words, the present invention provides compositions which can flow freely by selecting the correct carriers for guaranteeing the production of the compositions in an uninterrupted manner, the mechanical filling of the powder inhalator, a correct dosage and the release using powder inhalers.

The composition having ideal density and content ratio obtained by applied vacuum value is to guarantee the delivery of an exact dose of a composition to a patient by reaching target flow and NGI values.

According to a preferred embodiment of the present invention, a therapeutically effective amount of said pharmaceutical compositions is administered once and/or twice a day.

According to a preferred embodiment, the pharmaceutical compositions are used in the treatment of respiratory diseases selected from asthma and chronic obstructive pulmonary disease and other obstructive airways diseases. The combinations of the compounds according to the present invention are particularly useful in the treatment of respiratory diseases and conditions comprising asthma, acute respiratory failure, chronic pulmonary inflammatory disease, bronchitis, chronic bronchitis, chronic obstructive pulmonary disease, silicosis and similar conditions, as well as of immune disorders and conditions comprising allergic rhinitis and chronic sinusitis.

According to another embodiment, the pharmaceutical compositions are suitable for use in moisture-resistant blisters or capsules closed with safety barriers separately, successively or concurrently.

Blisters comprise aluminum particularly for the prevention of moisture intake and thus the fine particular fraction (FPF) of the pharmaceutical composition is maintained. Blisters are further closed with a moisture-safe barrier. Thus, water intake into the blister drug is prevented and the container is protected against external moisture intake.

According to a further preferred embodiment, the dry powder is within a capsule, wherein this capsule may be a pharmaceutically acceptable natural or synthetic polymer such as gelatin or hydroxypropyl methylcellulose.

25 mg doses are stored in air- and moisture-impermeable capsules and 5 mg doses in blisters.

It is also possible to use a pharmaceutically acceptable salt, solvate, polymorph or a racemic mixture of these active agents.

Fluticasone may be used in the form of a pharmaceutically acceptable salt, solvate, polymorph or a racemic mixture thereof, wherein the preferred form of fluticasone is fluticasone propionate or fluticasone furoate.

Salmeterol, in turn, may be used in the form of a pharmaceutically acceptable salt, solvate, polymorph or a racemic mixture thereof, wherein the preferred form of salmeterol is salmeterol xinafoate.

The compositions according to the present invention are placed to a dry powder inhaler comprising the blisters and a lid. The device comprises at least one lock mechanism, enabling the device to remain locked in two positions in which the device is ready for inhalation and the lid is in the closed position, and further enabling the device to setup again automatically, when the lid is closed.

Once the lid is opened, a force is exerted by the user to a trigger of the device. Then the trigger is guided by means of the guides provided in the device body and on the trigger itself and is slid into a slot. This action provides the operation of the mechanism. At the end of this sliding action, the trigger is coupled and immobilized so that a single dose released from the blister is administered through a mouthpiece. Keeping this slide-in or push-in action by the user until the locking position is activated ensures a complete peeling-off of the blister and an accurate administration of the required dosage amount. As a result of this locking effect, the trigger becomes immobilized and it remains out-of-use for a short period of time. This push-in action also causes a spring in the mechanism to become compressed between the trigger and the interior of device body. After the user administers the powder composition and closes the lid, the device is restored for the next use without requiring any user intervention by means of the mechanism comprised by the device.

If the compositions according to the present invention are used in a dry powder inhaler device via capsules, the capsules are loaded into the device by the user one-by-one and are administered by means of exploding the capsules.

## Claims

1. A filling method of an inhaler composition, **characterized in that** a composition
- having a bulk density ranging from 0.48 to 0.56 g/mL, a compressed density ranging from 0.78 to 0.92 g/mL, and a d(50) particle size ranging from 30 to 50 µm, and
- comprising 0.2 to 1.7% by weight of salmeterol with a d(50) particle size distribution ranging from 1 to 3 µm and 0.2 to 17.0% by weight of fluticasone with a particle size distribution ranging from 1 to 3 µm,
- 1 to 20% by weight of fine particle lactose with a d(50) particle size ranging from 2 to 10 µm, and 80 to 99% by weight of coarse particle lactose with a d(50) particle size ranging from 50 to 120 µm, is taken into a reservoir by applying a vacuum of 50000 Pa (500 mBar) to 8000 Pa (80 mBar) and then stopping the applied vacuum and transferring the composition into the blister cavity,
wherein the particle size measurement is conducted by laser diffraction and the particle size is measured by volume.

2. The filling method of a pharmaceutical composition according to claim 1, wherein said reservoir is provided on a cylinder.

3. The filling method according to any of the preceding claims, wherein said cylinder comprises additional reservoirs.

4. The filling method according to any of the preceding claims, wherein the composition taken into the reservoir under vacuum effect is filled in two parts into a blister or capsule.

5. The filling method according to any of the preceding claims, wherein the bulk density of the composition is preferably 0.50 - 0.55 g/mL.

6. The filling method according to any of the preceding claims, wherein the compressed density of the composition is preferably 0.80 - 0.90 g/mL.

7. The filling method according to any of the preceding claims, wherein the vacuum force is preferably between 40000 Pa (400 mBar) and 10000 Pa (100 mBar).

## Patentansprüche

1. Einfüllverfahren für eine Inhalatorzusammensetzung,
**dadurch gekennzeichnet, dass** die Zusammensetzung
- eine Schütteldichte im Bereich von 0,48 bis 0,56 g/mL, eine komprimierte Dichte im Bereich von 0,78 bis 0,92 g/mL und eine d(50) Partikelgröße im Bereich von 30 bis 50 µm hat, und
- aufweisend 0,2 bis 1,7 Gew% Salmeterol mit einer d(50) Partikelgrößenverteilung im Bereich von 1 bis 3 µm und 0,2 bis 7,0 Gew% Fluticasone mit einer Partikelgrößenverteilung im Bereich von 1 bis 3 µm aufweist,
- 1 bis 20 Gew% von feinpartikliger Laktose mit einer d(50) Partikelgröße im Bereich von 2 bis 10 µm und 80 bis 99 Gew% von gröberen Laktosepartikeln mit einer d(50) Partikelgröße im Bereich von 50 bis 120 µm in ein Reservoir durch Anlegen eines Vakuums von 50.000 Pa (500 mBar) bis 8.000 Pa (8 mBar) eingebracht wird und anschließendem Beenden des Anlegens von Vakuum und Transferieren der Komposition in den Blisterhohlraum,
wobei die Partikelgrößenmessung durch Laserdifraktion durchgeführt wird und die Partikelgröße über das Volumen gemessen wird.

2. Einfüllverfahren einer pharmazeutischen Zusammensetzung nach Anspruch 1, wobei das Reservoir auf einem Zylinder vorgesehen ist.

3. Einfüllverfahren nach einem der vorangehenden Ansprüche, wobei der Zylinder zusätzliche Reservoire aufweist.

4. Einfüllverfahren nach einem der vorhergehenden Ansprüche, wobei die Zusammensetzung, welche in das Reservoir unter Vakuumeffekt eingebracht ist, in zwei Teilen in einen Blister oder eine Kapsel gefüllt wird.

5. Einfüllverfahren nach einem der vorangehenden Ansprüche, wobei die Schütteldichte der Zusammensetzung bevorzugt 0,50 bis 0,55 g/mL beträgt.

6. Einfüllverfahren nach einem der vorangehenden Ansprüche, wobei die komprimierte Dichte der Zusammensetzung bevorzugt 0,80 bis 0,90 g/mL betragt.

7. Einfüllverfahren nach einem der vorangehenden Ansprüche, wobei die Vakuumkraft bevorzugt zwischen 40.000 Pa (400 mBar) und 10.000 Pa (100 mBar) ist.

## Revendications

1. Procédé de remplissage par une composition à inhaler, **caractérisé par le fait qu'**une composition
- ayant une masse volumique apparente se situant dans la plage de 0,48 à 0,56 g/mL, une masse volumique à l'état comprimé se situant dans la plage de 0,78 à 0,92 g/mL et une dimension de particule d(50) se situant dans la plage de 30 à 50 µm, et
- comprenant 0,2 à 1,7 % en poids de salmétérol ayant une distribution de la dimension de particule d(50) se situant dans la plage de 1 à 3 µm et 0,2 à 17,0 % en poids de fluticasone ayant une distribution de la dimension de particule se situant dans la plage de 1 à 3 µm,
- 1 à 20 % en poids de lactose en fines particules ayant une dimension de particule d(50) se situant dans la plage de 2 à 10 µm, et 80 à 99 % en poids de lactose en particules grossières ayant une dimension de particule d(50) se situant dans la plage de 50 à 120 µm, sont placés dans un réservoir par application d'un vide de 50 000 Pa (500 mBar) à 8000 Pa (80 mBar) puis arrêt du vide appliqué et transfert de la composition dans l'alvéole de blister,
dans lequel la mesure de la dimension de particule est conduite par diffraction laser et la dimension de particule est mesurée en volume.

2. Procédé de remplissage par une composition pharmaceutique selon la revendication 1, dans lequel ledit réservoir est disposé sur un cylindre.

3. Procédé de remplissage selon l'une quelconque des revendications précédentes, dans lequel ledit cylindre comprend des réservoirs supplémentaires.

4. Procédé de remplissage selon l'une quelconque des revendications précédentes, dans lequel la composition placée dans le réservoir sous effet de vide est introduite en deux parties dans un blister ou une capsule pour le remplissage.

5. Procédé de remplissage selon l'une quelconque des revendications précédentes, dans lequel la masse volumique apparente de la composition est, de préférence, de 0,50-0,55 g/mL.

6. Procédé de remplissage selon l'une quelconque des revendications précédentes, dans lequel la masse volumique à l'état comprimé de la composition est, de préférence, de 0,80-0,90 g/mL.

7. Procédé de remplissage selon l'une quelconque des revendications précédentes, dans lequel la force d'aspiration sous vide est, de préférence, entre 40 000 Pa (400 mBar) et 10 000 Pa (100 mBar).
